# EUROPÄISCHE PATENTANMELDUNG

(11) **EP 1 295 613 A1**
(43) Veröffentlichungstag der Anmeldung: **26.03.2003**
(21) Anmeldenummer: 02017172.4
(22) Anmeldetag: 31.07.2002
(51) Int. Cl.: A61L 9/12, B05B 11/04, A45D 34/02

(54) **Duftspender**

(30) Priorität: 30.08.2001 DE 20114352 U
(71) Anmelder: Klocke Verpackungs-Service GmbH, 76356 Weingarten (DE)
(72) Erfinder: Schührer, Herbert, 76646 Bruchsal (DE); Renner, Klaus, 76275 Ettlingen (DE)
(74) Vertreter: Frank, Gerhard, Dipl.-Phys.

(57) **Zusammenfassung**

Ein Duftspender weist einen Vorratsbehälter zur Aufnahme eines Aromaträgers auf, dessen Duftstoffe zumindest teilweise bei Druck auf den Vorratsbehälter durch eine Abgabeöffnung entweichen können. Der Vorratsbehälter kann die Form einer Flasche haben, wobei die vorzugsweise als Abbrechöffnung ausgebildete Abgabeöffnung im dünneren Bereich (Flaschenhals) angeordnet ist.

Vorzugsweise erstreckt sich ein Verpackungselement über den Abgabebereich des Vorratsbehälters, das dort derart ausgebildet ist, dass es mit einem Verschluss der Abgabeöffnung des Vorratsbehälters zusammenwirkt.

Vorratsbehälter oder Verpackungselement können als Standpackung ausgestaltet sein.

## Beschreibung

### Hintergrund der Erfindung

Der Mensch nimmt Dinge, Lebewesen und auch andere Menschen wesentlich auch durch seinen Geruchssinn wahr und sein Verhalten wird dadurch nachhaltig beeinflusst. Bereits geringe Konzentrationen von Duftstoffen können die Stimmung und somit das Verhalten des Menschen beeinflussen.

Duftspender machen sich diese biologischen Gegebenheiten zu nutze, sei es zur Schaffung einer allgemein angenehmen Atmosphäre, sei es auch zur bewussten kommerziellen Ausnutzung zur Erzielung eines bestimmten menschlichen Verhaltens, wie z.B. zur Unterstützung der Kauflust.

Diese stimulierende Wirkung gewinnt vor allem auch dann Bedeutung, wenn Entscheidungen von großer Tragweite getroffen werden sollen, wie bei wichtigen Konferenzen und Besprechungen.

### Stand der Technik

Es sind eine Vielzahl von technischen Lösungen von Verpackungen bekannt, um über einen längeren Zeitraum in einem bestimmten Volumen eine Duftstoffkonzentration zu erzeugen, die das gewünschte Verhalten fördern soll.

Einen gattungsgemäßen Duftspender zeigt die WO 00/06464. Hierbei ist in einem Vorratsbehälter ein poröses Material als Speicher für ein flüssiges Produkt enthalten. Bei Druck auf eine der Wandungen wird durch eine Öffnung ein Teil dieses Produktes als Spray oder Dispension an die Außenluft abgegeben.

Eine Verbesserung des ästhetischen Erscheinungsbildes eines derartigen Duftspenders versucht die WO 00/21853 dadurch zu erreichen, dass der Vorratsbehälter großflächig von einem banderolenähnlichen Verpackungselement umgeben ist, das Ausstanzungen und/oder Aufdrucke enthalten kann.

Aufgabe der Erfindung ist es, die Handhabung von Duftspendern der letztgenannten Art wesentlich zu verbessern.

Dieses wird durch die Erfindung gemäß den Merkmalen des Anspruchs 1 gelöst.

Ausgangspunkt der Erfindung ist ein saugfähiges Element, z.B. Schwamm, Filz, Karton in einer Tiefziehpackung als Vorratsbehälter. Dieses Element dient zur vollständigen Aufnahme eines Aromaträgermaterials (z.B. Parfümöl). In seinem Volumen ist dieses Element wesentlich kleiner als der von der Packung gebildete Raum, so dass sich in der Packung eine mit dem Duftstoff angereicherte Atmosphäre bilden kann. An geeigneter Stelle weist die Tiefzieh- oder Deckfolie eine kleine Öffnung auf, die z.B. durch ein Etikett, einen Klebepunkt oder einer abpeelbaren Folie verschlossen ist. Dieser Verschluss wird zur Aktivierung gelöst. Durch diese Öffnung kann die mit Duftstoffen angereicherte Luft vom Packungsinnern nach außen dringen. Durch Druck auf die Packung strömt verstärkt angereicherte Luft in die Umgebung. Die Ausformung der Tiefziehfolie ist so gestaltet, dass sie nach Entlastung wieder ihre ursprüngliche Form einnimmt. Das Luftvolumen im Innern der Packung nimmt wieder seine ursprüngliche Größe durch das Ansaugen von nicht angereicherter Luft an, die sich erneut mit Duftstoffen anreichern kann. Der Vorgang kann von neuem beginnen. Packungen auch ohne diese Rückstellfunktion sind möglich, hier wird die Abgabe von Duftstoffen durch die Größe der Austrittsöffnung und der somit veränderten Luftzirkulation gelöst.

Die Tiefziehpackung als Vorratsbehälter kann gemäss einer Ausgestaltung der Erfindung von einem Verpackungselement, vorzugsweise einer aus siegelfähigem Karton bestehenden Banderole, ummantelt sein. Diese Ummantelung dient zur optischen Optimierung, als Träger für Werbetexte und Funktionsbeschreibung und zur Unterstützung der technischen Funktion.

In weiterer Ausbildung kann vorgesehen sein, diese Display-Funktion der banderolenförmigen Umhüllung dadurch zu ergänzen und aufzuwerten, dass die Umhüllung auch technische Funktionalität bei der Aktivierung des Duftspenders erhält, insbesondere durch Zusammenwirken mit dem Verschluss der Tiefziehpackung.

### Kurze Beschreibung der Zeichnungen

Mehrere Ausführungsbeispiele werden anhand von Zeichnungen näher erläutert, es zeigen:
- Bild 1: Aufsicht und Schnitt eines ersten Ausführungsbeispiels des Duftspenders
- Bild 2: Aufsicht einer Variante des ersten Ausführungsbeispiels
- Bild 3: Aufsicht und Schnitt eines zweiten Ausführungsbeispiels
- Bild 4: Funktionsdarstellung des zweiten Ausführungsbeispiels
- Bild 5: Aufsicht und Schnitt eines dritten Ausführungsbeispiels
- Bild 6: Funktionsdarstellung des dritten Ausführungsbeispiels
- Bild 7: Aufsicht und Schnitt eines vierten Ausführungsbeispiels
- Bild 8: Funktionsdarstellung des vierten Ausführungsbeispiels
- Bild 9: Funktionsdarstellung eines fünften Ausführungsbeispiels
- Bild 10: Funktionsdarstellung eines sechsten Ausführungsbeispiels
- Bild 11: Perspektivische Darstellung eines siebten Ausführungsbeispiels
- Bild 12: Längsschnitt und Aufsicht der Tiefziehpackung des Duftspenders nach Bild 11 ohne Banderole,
- Bild 13: Längsschnitt und Aufsicht des Duftspenders nach Bild 11 mit Banderole,
- Bild 14: zwei Schnitte zur Funktion des Duftspenders nach Bild 11-13,
- Bild 15-18: Teilschnitte von Varianten des siebten Ausführungsbeispiels mit wieder verschließbaren Verschlüssen,
- Bild 19-20: Teilschnitte von Varianten mit nicht wieder verschließbaren Verschlüssen,
- Bild 21,22: Schnitte und perspektivische Darstellung eines achten Ausführungsbeispiels des Duftspenders als Abbrechtiefziehpackung, und
- Bild 23,24: Darstellungen von Ausgestaltungen des Verpackungselements.

### Beschreibung der Ausführungsbeispiele

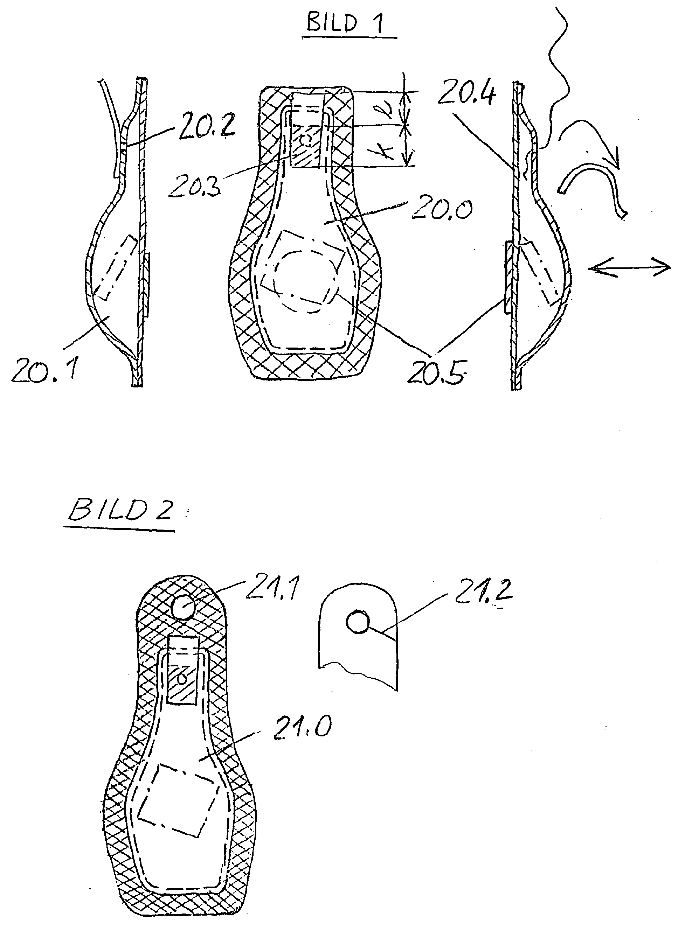

Bild 1 zeigt als erstes Ausführungsbeispiel einen Duftspender 20.0, sein Grundaufbau entspricht dem Aufbau der Tiefziehpackung 2.0 wie unter Bild 11 bis 13 beschrieben. Die Verwendung des Duftspenders 20.0 ohne zusätzlichs Verpackungselement als Umverpackung bietet einen grossen Spielraum für die Gestaltung des Tiefziehhofs 20.1, dieser kann einem Verkaufsprodukt wie z.B. einem Parfümflacon nachempfunden sein. Die Abgabeöffnung 20.2 für den Duftstoff der Tiefziehpackung 20.0 ist mittels eines Klebeetiketts 20.3 verschlossen. Das Klebeetikett 20.3 ist im Bereich K mit Kleber beschichtet, die einseitige Verlängerung L ist nicht mit Kleber beschichtet und dient als Abziehlasche. Die Öffnung 20.1 und das Etikett 20.2 können auch auf der flachen Deckfolie 20.4 angebracht sein. Zur Fixierung des Duftspenders z.B. auf einer Fliese ist auf dem Duftspender 20.0 an geeigneter Stelle ein Klebeetikett 20.5 angebracht.

Der Duftspender 21.0 nach der Variante gemäss Bild 2 ist in seinem Grundaufbau identisch mit dem Duftspender 20.0. Der Duftspender 21.0 ist in seinem Siegelbereich verlängert und weist dort eine Ausstanzung 21.1 auf, die das Aufhängen des Duftspenders ermöglicht. Die Ausstanzung 21.1 kann mit der Außenkontur des Duftspenders durch einen Schnitt 21.2 oder durch eine weitere Ausstanzung verbunden sein, die das Aufhängen an ein geschlossenes System ermöglicht.

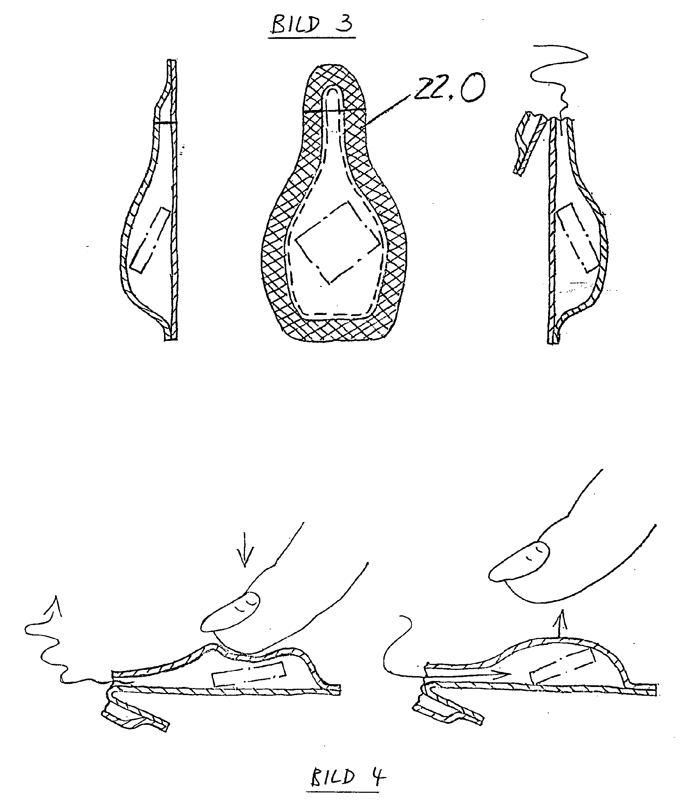

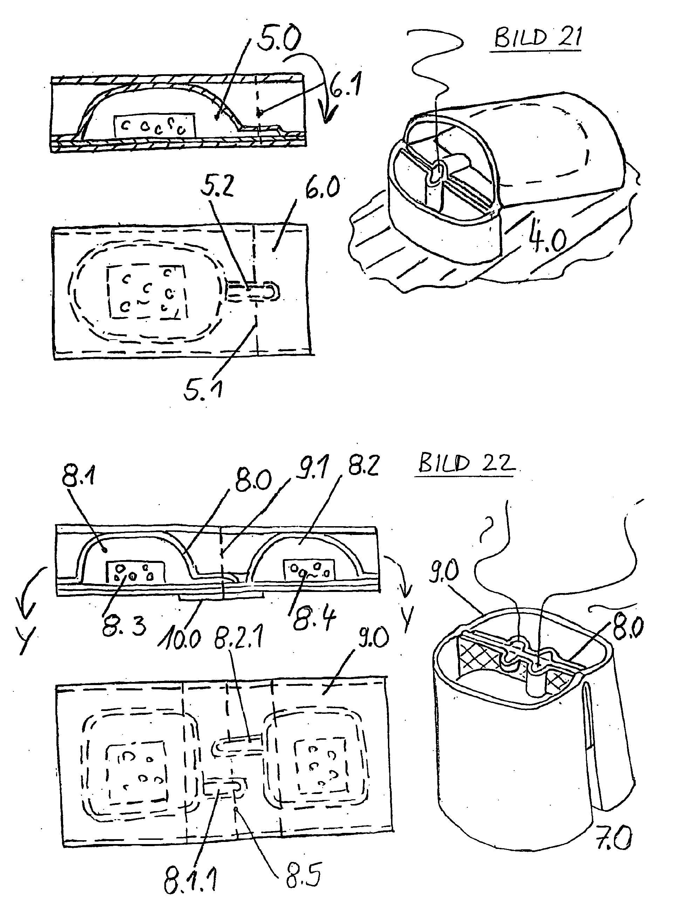

Bild 3 zeigt als zweites Ausführungsbeispiel einen Duftspender 22.0 als Abbrechtiefziehpackung wie unter Bild 21 beschrieben. Als Duftspender ohne Umverpackung bietet sie somit auch weitgehendsten Gestaltungsspielraum wie der unter Bild 1 beschriebene Duftspender 20.0.

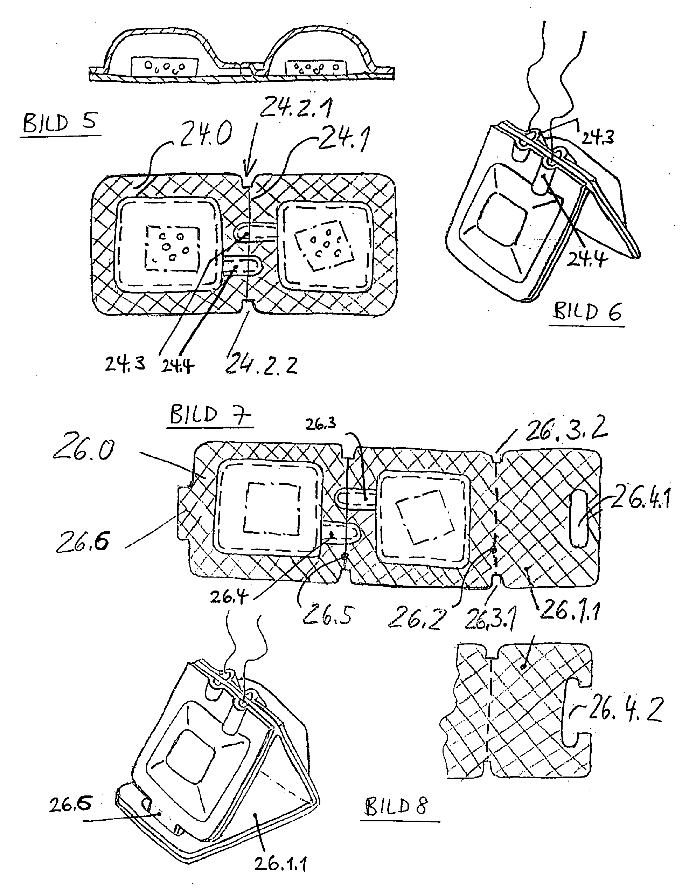

Bild 4 zeigt die Funktion der Abbrechtiefziehpackung 22.0; nach Abbrechen der Nase 22.1 strömt das Duftmedium aus, wie weiter unten zu Bild 11 erläutert.

Bild 5 zeigt als drittes Ausführungsbeispiel einen Duftspender 24.0 als Abbrechtiefziehpackung ohne Umverpackung mit 2 Tiefziehnäpfen. Der Grundaufbau entspricht der unter Bild 12 beschriebenen Packung 8.0. Zur Einleitung der Abbrechfunktion ist die Abbrechtiefziehpackung im Bereich der Rillung 24.1 mit Einkerbungen 24.2.1 und 24.2.2 versehen.

Bild 6 zeigt den Duftspender 24.0 nach Aufbrechen in aktiviertem und aufgestelltem Zustand.

Bild 7 zeigt als viertes Ausführungsbeispiel einen Duftspender 26.0. Sein Grundaufbau ist identisch mit dem des Duftspenders 24.0. Der Duftspender ist einseitig durch eine Lasche 26.1.1 verlängert, die durch eine Perforation, Rillung oder Prägelinie 26.2 gegenüber der eigentlichen Packung abgegrenzt ist. Die Perforation, Rillung oder Prägelinie 26.2 ist zur Erleichterung des Umbiegens mit Einkerbungen 26.3.1 und 26.3.2 versehen. Die Lasche 26.1.1 trägt randseitig eine Aussparung 26.4.1, die auch zur Außenkontur hin offen sein kann (26.4.2). Auf der gegenüberliegenden Seite weist der Duftspender 26.0 einen Ansatz 26.6 auf, der mittig zur Aussparung 26.4.1 bzw.26.4.2 sitzt. Die Aussparung 26.4.1 und 26.4.2 und der Ansatz 26.6 können sowohl auf Laschenseite wie auf Packungsseite angeordnet sein.

Bild 8 zeigt den Duftspender 26.0 in aktiviertem Zustand. Zum Aktivieren wird der Duftspender entlang seiner Rillung 26.5 aufgebrochen; die Lasche 26.1.1 wird um ihre Perforationsachse gebogen und in die Aussparung 26.4.1 bzw. 26.4.2 eingerastet, so daß sich eine Dreiecksstruktur mit gutem Stand ergibt.

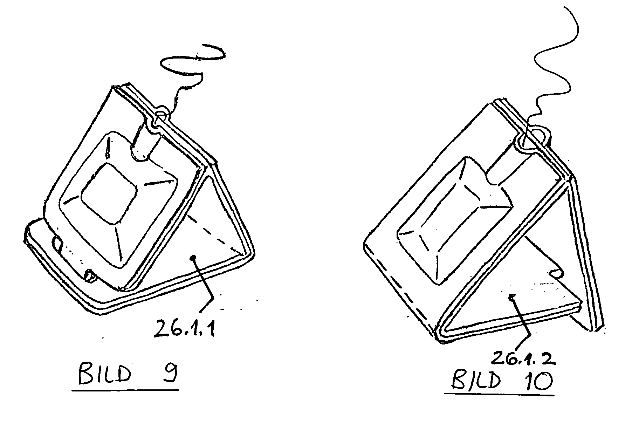

Das fünfte Ausführungsbeispiel des Duftspender 26.0 nach Bild 9 ist nur mit einem Tiefziehnapf ausgestattet. Der Bereich zwischen Rillung 26.5 und Perforation 26.2 dient somit nur als Standhilfe. Der Tiefziehnapf kann auch im Bereich zwischen Rillung 26,5 und Perforation 26.2 angeordnet sein.

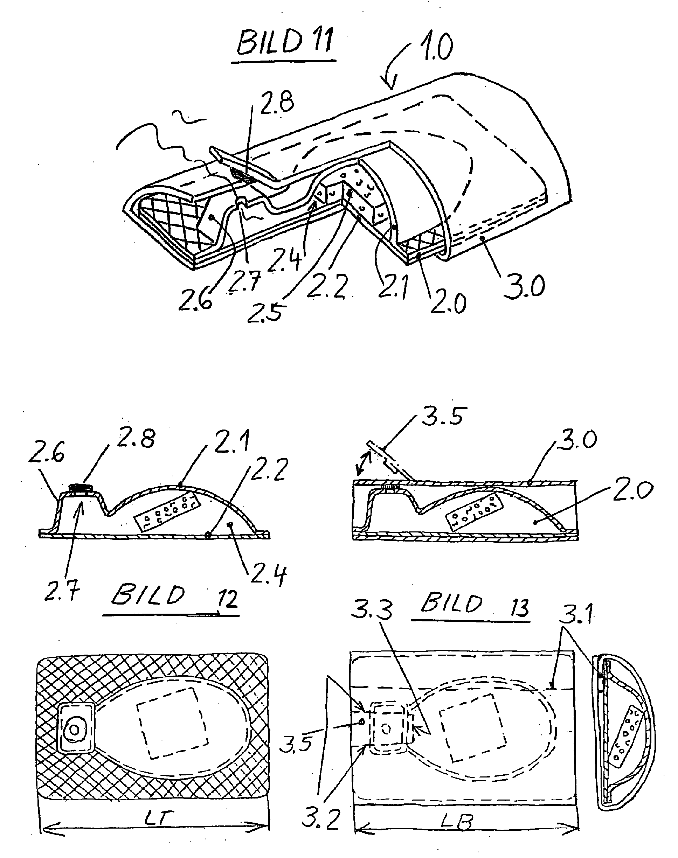

In Bild 10 ist diese Variante als sechstes Ausführungsbeispiel mit getauschter Lage von Aussparung 26.4 und Ansatz 26.6 dargestellt.

Bei den im folgenden beschriebenen weiteren Ausführungsbeispielen ist der Duftspender von einem zusätzlichen Verpackungselement als Umverpackung umgeben:

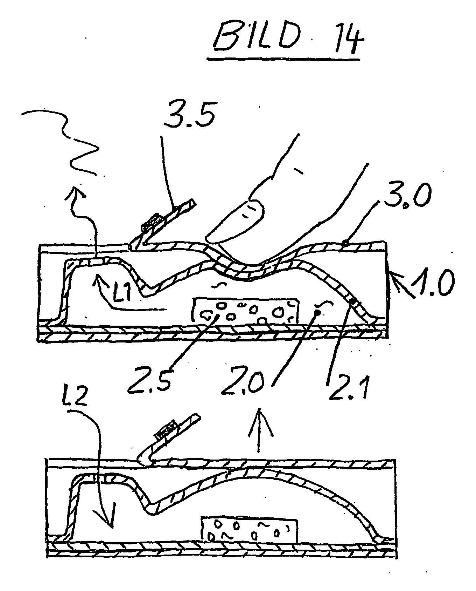

Bild 11 zeigt als siebtes Ausführungsbeispiel einen Duftspender 1.0 in perspektivischer Darstellung in aktiviertem Zustand. In Bild 12 ist die Tiefziehpackung 2.0 im Vollschnitt und in Draufsicht in Bild 13 die Komplettpackung in drei Ansichten dargestellt.

Der Duftspender 1.0 setzt sich aus einer Tiefziehpackung 2.0 als Vorratsbehälter und einem Verpackungselement in Form einer Banderole 3.0 zusammen. Die Tiefziehpackung 2.0 besteht aus einer thermoplastisch verformten Tiefziehfolie 2.1 und einer Deckfolie 2.2. Die Tiefziehfolie 2.1 ist so geformt, dass sie nach Deformation zur Abgabe des Duftstoffes wieder ihre Ausgangsform einnimmt. Auf die Tiefziehfolie 2.1 ist die Deckfolie 2.2 gasdicht aufgesiegelt und bildet einen Hohlraum 2.4. In den Hohlraum 2.4 ist ein saugfähiges Element 2.5 eingebracht, das vor dem Aufsiegeln der Deckfolie 2.2 auf die Tiefziehfolie 2.1 mit einem flüssigen Duftträger benetzt wird. Die Luft im Hohlraum 2.4 reichert sich mit den Duftmolekülen, die dem saugfähigen Element 2.5 entweichen, an. Die Tiefziehpackung 2.0 ist zu einer Seite hin durch einen Ansatz 2.6 verlängert. Der Ansatz 2.6 ist von einer Austrittsöffnung 2.7 durchbrochen und bis zum Aktivieren durch ein Verschlusselement wie z.B. einen Klebepunkt 2.8 oder mittels eines (hier nicht dargestellten) Etiketts verschlossen.

Die Tiefziehpackung 2.0 ist von der Banderole 3.0 (vorzugsweise aus Papier oder Karton) umschlossen, wobei der Randbereich im Bereich der Überlappung 3.1 (Bild 13) der Banderole 3 siegelfähig ist. Die Verbindung im Bereich der Überlappung 3.1 kann sowohl durch Siegeln wie auch durch Kleben hergestellt sein. Das Maß LT der Tiefziehpackung 2.0 ist der Regel gleich oder kleiner als das Maß LB der Banderole 3.0, so dass die Tiefziehpackung 2.0 nicht übersteht.

Insoweit ist der grundsätzliche Aufbau bei den dargestellten Ausführungsbeispielen weitgehend identisch. Die Ausführungsbeispiele unterscheiden sich in der Gestaltung des Öffnungsbereichs im Zusammenwirken zwischen Banderole und Tiefziehpakkung, wie dies im folgenden erläutert wird:

Im Bereich der verschlossenen Austrittsöffnung 2.7 hat die Banderole 3.0 bei diesem Ausführungsbeispiel zwei Perforationen 3.2 parallel zur Längsachse der Packung.

Die Perforationen 3.2 sind an ihrem Ende durch eine weitere Perforation oder Prägelinie 3.3 verbunden, die rechtwinklig zur Längsachse verläuft.

Um den Duftspender 1.0 zu aktivieren, wird die Banderole 3.0 entlang der Perforationen 3.2 aufgerissen. Die Prägelinie 3.3 dient dabei als Scharnier. Der Kleber des klebepunktes 2.8 (bzw. des Etiketts) ist so gewählt, dass die Haftung an der Banderole 3.0 größer ist als die Haftung an der Tiefziehpackung 2.0. Nach dem Hochklappen des Mittelbereichs 3.5 der Banderole 3.0 ist somit der Duftspender geöffnet.

Ist der Duftspender 1.0 geöffnet, kann durch Druck auf die Banderole 3.0 das Austreten der aromatisierten Luft L1 verstärkt werden. Nach Entlastung nimmt die Tiefziehfolie 2.1 der Tiefziehpackung 2.0 wieder ihre ursprüngliche Form ein, nicht aromatisierte Luft L2 kann in die Tiefziehpackung 2.0 einströmen. Zum Wiederverschließen wird der Mittelbereich 3.5 wieder gegen die Tiefziehpackung 2.0 gedrückt. Der Vorgang kann erneut beginnen.

Bild 15 zeigt eine erste Variante des siebten Ausführungsbeispiel des Verschlussbereichs, ähnlich der unter Bild 11-13 beschriebenen Variante. Zur besseren Handhabung ist hier der Mittelbereich 3.5 zwischen den Perforationslinien zu einer überstehenden Lasche ausgebildet.

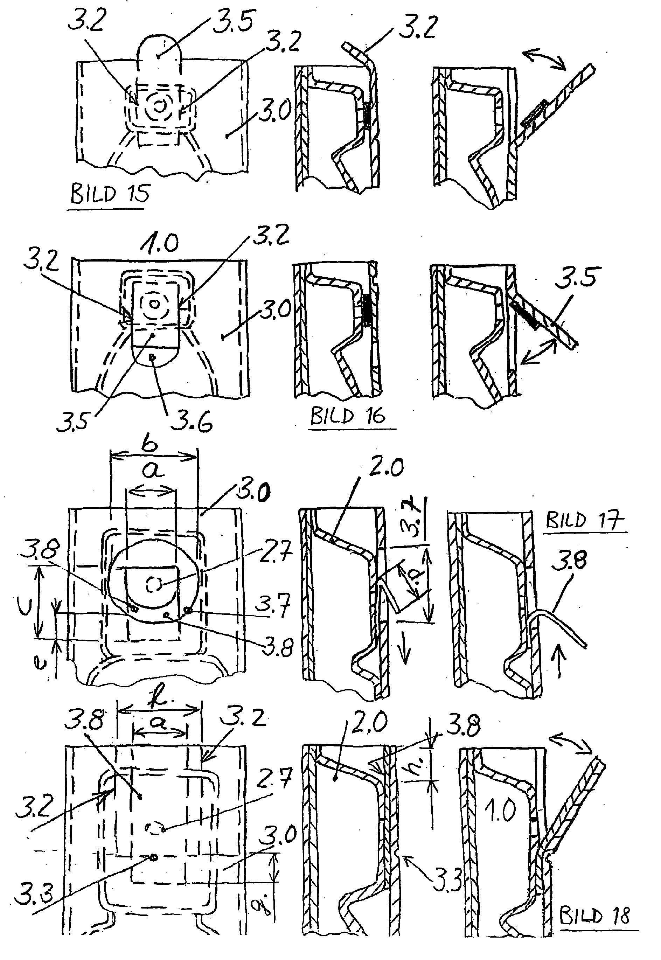

Bei der zweiten Variante gemäß Bild 16 ist die Lasche 3.5 der Banderole 3.0 nach innen verlegt, zum besseren Greifen der Lasche 3.5 ist im unten anschließenden Bereich eine Aussparung 3.6 freigestanzt. Die Seiten der Lasche 3.2 können sowohl gestanzt als auch perforiert sein. Zum Öffnen des Duftspenders 1.0 wird die Lasche 3.5 durch die Aussparung 3.6 gegriffen und nach oben gezogen. Zum Verschließen der Packung 1.0 wird die Lasche 3.5 wieder in ihre Ausgangslage zurückgedrückt.

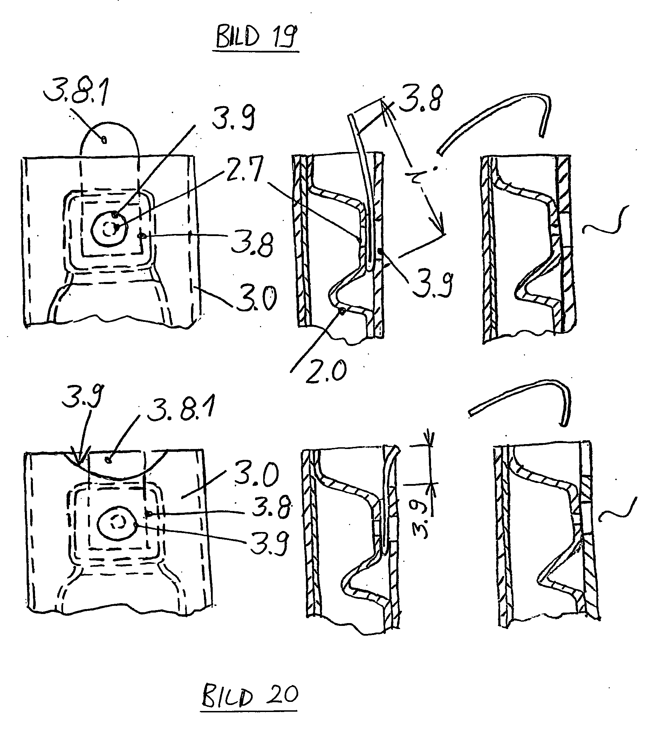

Beim der dritten Variante nach Bild 17 hat die Banderole 3.0 im Bereich der Austrittsöffnung 2.7 der Tiefziehpackung 2.0 eine Lochstanzung 3.7. Die Austrittsöffnung 2.7 ist mittels eines Klebeetiketts 3.8 als Verschluss abgedeckt, wobei das Maß a des Klebeetiketts 3.8 kleiner ist als das Maß b der Stanzung 3.7. Der Bereich c des Klebeettikettes 3.8 ist auf der Berührungsfläche mit der Tiefziehpackung 2.0 mit Kleber beschichtet. Der Bereich d des Klebeettikettes 3.8 ist kleberfrei und zu einer Lasche ausgebildet, die umgelegt sein kann. Das Klebeetikett ragt im Bereich e unter die Banderole 3.0, so dass sich beim Lösen des Etiketts eine Sperr ergibt. Zum Verschließen wird das Klebeetikett 3.8 wieder zurückgeschoben.

Beim vierten Variante nach Bild 18 ist die Austrittsöffnung 2.7 der Tiefziehpackung 2.0 mittels eines Klebeetiketts 3.8 verschlossen. Im Bereich des Klebeetiketts 3.8 hat die Banderole 3.0 zwei Perforationslinien 3.2, wobei der Perforationsabstand f größer ist als die Breite a des Klebeetiketts 3.8. Die Perforationslinien 3.2 sind durch eine Perforation oder Prägelinie 3.3 verbunden. Diese Prägelinie 3.3
dient als Scharnier. Das Klebeetikett 3.8 ragt um das Maß g über die Prägelinie 3.3 hinaus und kann somit nicht vollständig abgelöst werden. Der Griffbereich h des Klebeetiketts 3.8 ist kleberfrei. Zum Öffnen wird der Bereich zwischen den beiden Perforationslinien 3.2 mit dem Klebeetikett 3.8 gehalten und entlang der Perforationslinien 3.2 bis zur Prägelinie 3.3 aufgerissen und somit der Duftspender geöffnet. Zum Verschließen wird der aufgerissene Bereich wieder in seine Ausgangslage zurück gedrückt.

Bild 19 zeigt als fünfte Variante eine nicht wiederverschließbare Öffnungsvariante des Duftspenders 1.0. Die Austrittsöffnung 2.7 der Packung 2.0 ist mit einem Klebeetikett 3.8 verschlossen. Im Bereich der Austrittsöffnung 2.7 weist die Banderole 3.0 eine Lochstanzung 3.9 auf, die etwas größer ist als die Austrittsöffnung 2.7 der Tiefziehpackung 2.0. Der kleberfreie Bereich i des Klebeetiketts ist umgefalzt und ragt über das Ende der Packung hinaus. Das Klebeetikett 3.8 kann durch Zug an seiner Peellasche 3.8.1 abgezogen werden.

Eine sechste Variante nach Bild 20 beinhaltet im wesentlichen die Merkmale, wie sie unter Bild 19 beschrieben wurden. Die Peellasche 3.8.1 steht allerdings nicht über die Packung 1.0 hinaus. Zum besseren Greifen der Peellasche 3.8.1 ist in diesem Bereich die Banderole 3.0 eine randseitige Aussparung 3.9 vorgesehen.

Bild 21 zeigt als achtes Ausführungsbeispiel einen Duftspender 4.0; Grundlage ist eine Abbrechtiefziehpackung 5.0, wie sie schon für andere Produkte im Einsatz ist; sie wird deshalb nicht näher beschrieben. In der Abbrechtiefziehpackung 5.0 ist ebenfalls ein mit flüssigem Duftträger benetztes Element eingebracht, wie unter Bild 11-14 beschrieben. Der Vorratsbehälter 5.0 ist ebenfalls von einer Banderole umgeben, deren Grundaufbau unter Bild 11-13 beschrieben ist. Im Bereich der Sollbruchstelle 5.1, die über die Abbrechnase 5.2 der Abbrechtiefziehpackung 5.0 verläuft, hat die Banderole 6.0 eine umlaufende Perforation 6.1.

Zum Aktivieren wird die Banderole 6.0 mit der darin liegenden Abbrechtiefziehpakkung 5.0 in Pfeilrichtung z aufgebrochen.

Bild 22 zeigt eine Variante des Duftspenders wie unter Bild 21 beschrieben mit einem Vorratsbehälter als Abbrechtiefziehpackung 8.0 mit zwei Tiefziehhöfen 8.1 und 8.2 jeweils bestückt mit einem saugfähigen Element 8.3 und 8.4 zur Aufnahme von gleichen oder verschiedenen Aromaträgern. Zur Mitte hin sind zwei Abbrechnasen 8.1.1 und 8.1.2 versetzt zueinander angeordnet. Mittig über die Abbrechtiefziehpackung 8.0 und deren Abbrechnasen 8.1.1 und 8.2.1 verläuft eine Rillung 8.5 als Sollbruchstelle. Die Abbrechtiefziehpackung 8.0 ist ebenfalls von einer Banderole 9.0 umgeben. Mittig und somit über der Abbrechrillung 8.5 der Tiefziehpackung 8.0 liegend, weist die Banderole 9.0 eine umlaufende Perforation 9.1 auf. An der Unterseite der Packung 7.0 ist ein Klebeetikett 10.0 angebracht, wobei die der Packung abgewandte Seite nur an sich selber haftet. Zum Aktivieren werden die beiden Packungsenden in Pfeilrichtung y gebogen und somit Banderole 9.0 und Abbrechtiefziehpakkung 8.0 aufgebrochen. Das ebenfalls zusammengeführte Klebeetikett 10.0 verbindet sich und hält die geknickte Packung 7.0 in Position; diese kann somit gut auf ihren Stirnflächen aufgestellt werden. Durch die aufgebrochenen Abbrechnasen 8.1.1 und 8.2.1 können die beiden Aromen entweichen und sich mischen.

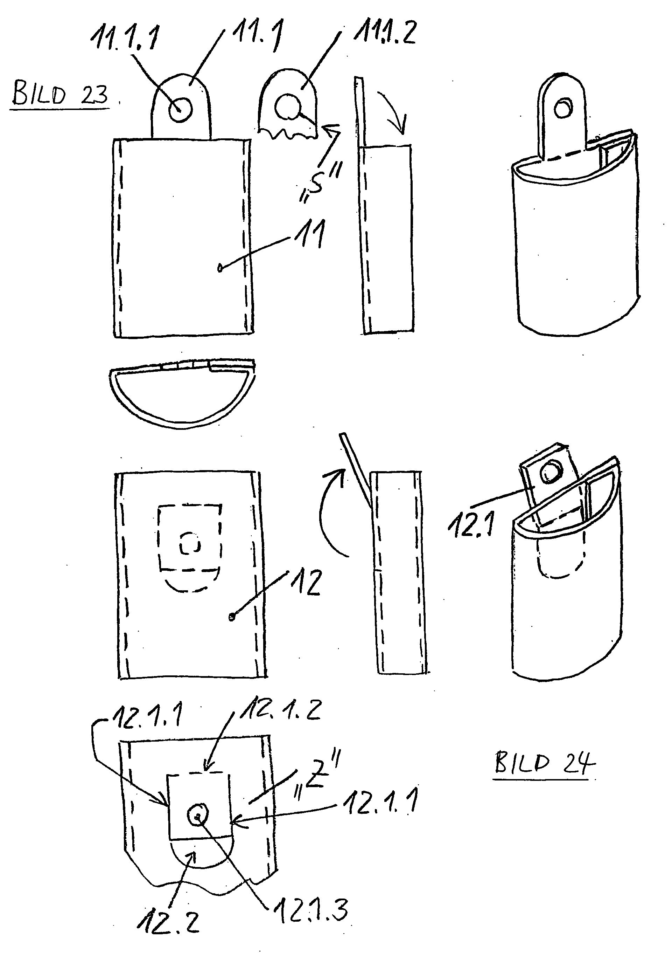

Bild 23 und 24 zeigen Weiterbildungen des Duftspenders außerhalb des Öffnungsbereichs:

Die Banderole 11 ist durch eine Lasche 11.1 verlängert (Bild 13). Die Lasche 11.1 hat eine Lochung 11.1.1 und bietet somit eine Möglichkeit zum Aufhängen der Pakkung. In Darstellung 11.1.2 ist die Lasche 11.1 mit einem zusätzlichen Stanzschnitt s zur Erleichterung des Einhängens versehen.

In der Variante nach bild 24 ist die Lasche 12.1 auf der Rückseite der Packung integriert. Darstellung Z zeigt die Banderole 12 von hinten. Die Seitenform 12.1.1 der Lasche 12.1 ist perforiert und vorgestanzt. Die beiden Seitenperforationen 12.1.1 sind an ihrem oberen Ende durch eine weitere Perforation oder Prägekante 12.1.2 verbunden, die vorperforierte Lasche 12.1 hat eine Lochstanzung 12.1.3, die das spätere Aufhängen ermöglicht. Im Bereich des späteren Laschenendes ist eine Lochstanzung 12.2 angebracht, sie soll das Angreifen der Lasche erleichtern. Zum Aufhängen wird über die Stanzung 12.2 die Lasche 12.1 gegriffen und nach hinten über die Prägekante 12.1.2 nach oben gebogen. Die umgebogene Lasche 12.1 ragt über die Banderole 12 hinaus und bietet somit eine Aufhängemöglichkeit.

## Patentansprüche

**1.** Duftspender mit einem Vorratsbehälter zur Aufnahme eines Aromaträgers, dessen Duftstoffe zumindest teilweise bei Druck auf den Vorratsbehälter durch eine Abgabeöffnung entweichen können.

**2.** Duftspender nach Anspruch 1, **dadurch gekennzeichnet, dass** er die Form einer Flasche hat, wobei die vorzugsweise als Abbrechöffnung ausgebildete Abgabeöffnung im dünneren Bereich (Flaschenhals) angeordnet ist.

**3.** Duftspender nach Anspruch 1, **dadurch gekennzeichnet, dass** der Vorratsbehälter eine Tiefziehpackung (2.0) ist, bestehend aus einem Tiefziehteil (2.1) aus thermoplastisch verformter Kunststofffolie und einer auf diese aufgesiegelte Deckfolie (2.2).

**4.** Duftspender nach Anspruch 1, **dadurch gekennzeichnet, dass** am Verpackungselement eine Aufhängelasche (11.1, 12.1) an- oder ausgeformt ist.

**5.** Duftspender nach Anspruch 1, **dadurch gekennzeichnet, dass** der Vorratsbehälter aus zwei Tiefziehnäpfen (8.1,8.2) besteht, deren Abbrechnasen (8.1.1, 8.2.1) sich unter einer gemeinsamen Perforation oder Rillung (8.5) erstrecken.

**6.** Duftspender nach Anspruch 1, **dadurch gekennzeichnet, dass** der Aromaträger im Abstand von der Abgabeöffnung (2.7) im Vorratsbehälter gehalten ist.

**7.** Duftspender nach Anspruch 3, **dadurch gekennzeichnet, dass** das Tiefziehteil im Siegelbereich mindestens eine Rillung (24.1, 26.2, 26.5) aufweist, die derart durch mindestens eine Abbrechnase (24.3, 24.4) verläuft/verlaufen, dass nach Abbiegung eine zusammenhängende Standpackung mit freier/freien Abgabeöffnung(en) entsteht.

**8.** Duftspender nach Anspruch 7, **dadurch gekennzeichnet, dass** beidseitig der Rillung (24.1) ein Abschnitt mit einer Tiefziehkammer liegt, so dass nach dem Abbrechen zwei freie Abgabeöffnungen entstehen.

**9.** Duftspender nach Anspruch 8, **dadurch gekennzeichnet, dass** eine Rillung (26.5) sowie eine Perforation, Rillung oder Prägelinie (26.2) vorgesehen sind, so dass eine Verbindungslasche (26.1.1) oder Standfläche (26.1.2) gebildet wird, die mit dem gegenüberliegenden Abschnitt zu einer Standpackung mit etwa dreieckigem Profil verbindbar ist.

**10.** Duftspender nach Anspruch 9, **dadurch gekennzeichnet, dass** die beiden Endabschnitt eine Aussparung (26.4.1, 26.4.2) und einen korrespondierenden Ansatz (26.6) zur Fixierung der Standpackung aufweisen.

**11.** Duftspender nach Anspruch 1, **dadurch gekennzeichnet, dass** sich ein Verpakkungselement über den Abgabebereich des Vorratsbehälters erstreckt und dort derart ausgebildet ist, dass es mit einem Verschluss der Abgabeöffnung des Vorratsbehälters zusammenwirkt.

**12.** Duftspender nach Anspruch 11, **dadurch gekennzeichnet, dass** im Bereich der Abgabeöffnung (2.7) ein diese überdeckendes Verschlusselement (2.8) angeordnet ist, das mit der Unterseite einer Betätigungseinrichtung des Verpackungselements derart verbunden ist, dass beim Öffnen der Betätigungsvorrichtung das Verschlusselement (2.8) an dieser haften bleibt und die Abgabeöffnung (2.7) freigibt.

**13.** Duftspender nach Anspruch 12, **dadurch gekennzeichnet, dass** die Betätigungseinrichtung als eine durch Perforationslinien (3.2) des Verpackungselements definierte Lasche (3.5) ausgebildet ist.

**14.** Duftspender nach Anspruch 13, **dadurch gekennzeichnet, dass** die Lasche (3.5) über eine Kante des Verpackungselements hinausragt.

**16.** Duftspender nach Anspruch 11, **dadurch gekennzeichnet, dass** im Verpackungselement eine die Abgabeöffnung (2.7) einbeziehende Aussparung (3.7) vorgesehen ist, in die ein zwischen Verpackungselement und Vorratsbehälter gehaltener, insbesondere verschiebbarer Verschluss hineinragt.

**17.** Duftspender nach Anspruch 12, **dadurch gekennzeichnet, dass** der Verschluss ein Klebeetikett (3.8) ist.

**18.** Duftspender nach Anspruch 16 und 17, **dadurch gekennzeichnet, dass** das Klebeetikett (3.8) über eine Kante des Verpackungselements hinausragt und von dort zwischen Verpackungselement und Vorratsbehälter herausziehbar ist.

**19.** Duftspender nach Anspruch 16 und 17, **dadurch gekennzeichnet, dass** das Klebeetikett (3.8) in eine randseitige Aussparung (3.9) des Verpackungselements hineinragt und von dort herausziehbar ist.

**20.** Duftspender nach Anspruch 11, **dadurch gekennzeichnet, dass** das Verpakkungselement eine über eine Abbrechnase (5.2) des Vorratsbehälters verlaufende Perforation (5.1) aufweist, bei deren Aufreißen die Abbrechnase (5.2) geöffnet wird.

**21.** Duftspender nach Anspruch 12, **dadurch gekennzeichnet, dass** das Verpakkungselement eine Banderole (3.0) ist, die den Vorratsbehälter umschließt.

**22.** Duftspender nach Anspruch 20 und 21, **dadurch gekennzeichnet, dass** die Banderole (3.0) einen Überlappungsbereich (3.1) im Bereich der Deckfolie (2.2) der Tiefziehpackung (2.0) aufweist, der klebe- oder siegelfähig ist.

**23.** Duftspender nach Anspruch 21, **dadurch gekennzeichnet, dass** die Banderole (3.0) aus einem siegelfähigen Kartonzuschnitt gebildet ist.
